# EUROPÄISCHE PATENTSCHRIFT

(11) **EP 0 402 743 B1**
(45) Veröffentlichungstag und Bekanntmachung des Hinweises auf die Patenterteilung: **14.09.1994**
(21) Anmeldenummer: 90110650.0
(22) Anmeldetag: 06.06.1990
(51) Int. Cl.: C07D 303/28, B01J 23/46

(54) **Verfahren zur Herstellung von Di-(p-glycidoxicyclohexyl)-methan**
Process for the production of di-(p-glycidoxycyclohexyl)-methane
Procédé pour la préparation de di-(glycidoxycyclohexyl)-méthane

(30) Priorität: 13.06.1989 DE 3919228
(43) Veröffentlichungstag der Anmeldung: 19.12.1990
(73) Patentinhaber: BASF Aktiengesellschaft, 67063 Ludwigshafen (DE)
(72) Erfinder: Schuster, Ludwig, Dr., D-6703 Limburgerhof (DE)

(56) Entgegenhaltungen:
- EP-A- 0 108 720
- DE-A- 3 629 632
- DE-B- 1 518 119

## Beschreibung

Die vorliegende Erfindung betrifft ein neues und verbessertes Verfahren zur Herstellung von Di-[p-glycidoxicyclohexyl]-methan durch katalytische Hydrierung von Di-[p-glycidoxi-phenyl]-methan in Gegenwart eines Rutheniumkatalysators bei erhöhten Temperaturen und erhöhten Drücken.

Aus der US-A 3,336,241 ist bekannt, daß man im Di-[p-glycidoxi-phenyl]-methan an Rhodium und Ruthenium auf Trägern den Aromaten selektiv hydrieren kann.

Aus der DE-A-3,629,632 ist ein Verfahren zur Herstellung von 2,2-Di-[p-glycidoxicyclohexyl]-propan durch katalytische Hydrierung von 2,2-Di-[-p-glycidoxi-phenyl]-propan in Gegenwart eines Rutheniumkatalysators bekannt, der weitgehend selektiv verläuft.

Vergleicht man in der US-A-3,336,241 das Beispiel 2 mit dem Beispiel 3 so zeigt sich, daß die Hydrierung unterschiedlich abläuft, je nachdem ob man von dem Diphenylpropan- oder dem Diphenylmethanderivat ausgeht. Beim Diphenylmethanderivat benötigt man weniger Katalysator und erzielt eine geringere Selektivität.

Es bestand daher die Aufgabe, die Reaktion der DE-A-3,629,632 auf das Di-[-p-glycidoxi-phenyl]-methan zu übertragen.

Demgemäß wurde ein neues und verbessertes Verfahren zur Herstellung von Di-[p-glycidoxicyclohexyl]-methan durch katalytische Hydrierung gefunden, welches dadurch gekennzeichnet ist, daß man Di[p-glycidoxi-phenyl] methan in Gegenwart von 0,05 bis 10 Gew.% Ruthenium eines Rutheniumkatalysators bei Temperaturen im Bereich von 30 bis 60°C und Drücken oberhalb von 100 bar hydriert. Die Hydrierung des Di-[p-glycidoxi-phenyl]-methans zum Di-[p-glycidoxi-cyclohexyl]-methan gelingt selektiv, wenn man ein nicht desaktiviertes Rutheniumoxidhydrat, das durch Fällung aus einer wäßrigen Lösung von Rutheniumtrichlorid gewonnen worden ist, einsetzt.

Man verwendet 0,05 bis 10 Gew.-%, vorzugsweise 0,05 bis 1 Gew.-% besonders bevorzugt 0,05 bis 0,30 Gew.-% Ruthenium bezogen auf das Di-[p-glycidoxi-phenyl]-methan. Im allgemeinen reichen zwischen 0,05 bis 0,15 Gew.-% Ruthenium, um eine praktisch quantitative Hydrierung, d.h. bis zum Ende der Wasserstoffaufnahme, zu erreichen. Die Oxiranringe werden praktisch nicht angegriffen.

Besonders auffällig ist hierbei die starke Abnahme der Viskosität beim Übergang vom Diphenylmethangerüst zum Di-cyclohexylmethangerüst, die man nach dem Abdestillieren des Lösungsmittels beobachtet. So fällt beispielsweise die Viskosität von etwa 20 000 mPas·s auf ca. 1800 bei 20°C; dies bedeutet eine Abnahme auf nur 6 % des ursprünglichen Wertes. Diese Eigenschaft ist von besonderem Wert für die Technik der Epoxidlacke und Harze.

Die Hydrierung wird bei Drücken oberhalb 100 bar bis 1000 bar durchgeführt; im allgemeinen werden Drücke von 200 bis 325 bar angewendet.

Wegen der hohen Viscosität der Ausgangssubstanz ist es besonders zweckmäßig, die Hydrierung in Lösung durchzuführen. Als Lösungsmittel werden Ether bevorzugt wie Glykoldimethylether oder Methyl-tert.-butylether, besonders bevorzugt Tetrahydrofuran und Dioxan eingesetzt. Es kann auch in sehr konzentrierten Lösungen gearbeitet werden, beispielsweise in einer 50%igen Lösung in Tetrahydrofuran.

Bei der Auswahl der Reaktionstemperatur sind verhältnismäßig enge Grenzen gesetzt. So werden bei höheren Temperaturen bereits sehr weitgehend die Oxiranringe geöffnet, bei zu niedrigen Temperaturen hingegen die aromatischen Ringe noch nicht abgesättigt. Der schmale Temperaturbereich, der für die selektive Kernhydrierung bleibt, liegt zwischen 30 und 60°C, bevorzugt zwischen 40 und 50°C.

Für die Hydrierung muß nicht das reine Di-[p-glycidoxi-phenyl]-methan vorliegen. Man kann auch kondensationsprodukte hydrieren, die einen bestimmten Anteil, z.B. bis 30 %, an höherkondensierten Produkten enthalten.

### Beispiel

In einem mit einem Magnetrührer versehenen Autoklav von 50 l Inhalt werden 12 kg des Polyglycidylethers eines Phenol-Formaldehyd-Kondensates in 12 kg Tetrahydrofuran gelöst. Zu dieser Lösung werden 30 g Rutheniumoxidhydrat mit einem Gehalt von 12 g Ruthenium hinzugegeben. Die Hydrierung wird unter einem Druck von 300 bar bei einer Temperatur von 50°C ausgeführt. Die Wasserstoffaufnahme endete nach 27 Stunden.

Der Hydrieraustrag wird abfiltriert und durch Aktivkohle völlig entfärbt. Anschließend destilliert man i.Vak. das Lösungsmittel ab. Man erhält 12,3 kg farbloses Di-[p-glycidoxi-cyclohexyl]-methan mit einer Viscosität bei 20°C von 1800 m·Pa·s.

## Patentansprüche

1. Verfahren zur Herstellung von Di-[p-glycidoxicyclohexyl]-methan durch katalytische Hydrierung, dadurch gekennzeichnet, daß man Di-[p-glycidoxi-phenyl]-methan in Gegenwart von 0,05 bis 10 Gew.-% Ruthenium eines Rutheniumkatalysators bei Temperaturen im Bereich von 30 bis 60°C und Drücken oberhalb von 100 bar hydriert.

## Claims

1. A process for preparing di-[p-glycidoxycyclohexyl]methane by catalytic hydrogenation, which comprises hydrogenating di-[p-glycidoxyphenyl]methane in the presence of from 0.05 to 10 % by weight of ruthenium of a ruthenium catalyst in the range from 30 to 60°C and at above 100 bar.

## Revendications

1. Procédé de préparation du di-[p-glycidoxycyclohexyl]-méthane par hydrogénation catalytique, caractérisé en ce que l'on hydrogène le di-[p-glycidoxyphényl]-méthane en présence de 0,05 à 10% en poids de ruthénium d'un catalyseur au ruthénium, à des températures qui varient de 30 à 60°C et sous des pressions supérieures à 100 bars.
